(19) Europäisches Patentamt
European Patent Office
Office européen des brevets

(11) Veröffentlichungsnummer: **0 217 371**
**A2**

(12) # EUROPÄISCHE PATENTANMELDUNG

(21) Anmeldenummer: 86113482.3

(22) Anmeldetag: 01.10.86

(51) Int.Cl.⁴: C 07 F 9/65
C 12 Q 1/42

(30) Priorität: 01.10.85 DE 3534927

(43) Veröffentlichungstag der Anmeldung: 08.04.87 Patentblatt 87/15

(84) Benannte Vertragsstaaten: AT BE CH DE ES FR GB GR IT LI LU NL SE

(71) Anmelder: Boehringer Mannheim GmbH
Sandhofer Strasse 116
D-6800 Mannheim 31(DE)

(72) Erfinder: Mühlegger, Klaus
Römerstrasse 7
D-8121 Polling(DE)

(72) Erfinder: von der Eltz, Herbert, Dr. rer. nat.
Kerschensteinerstrasse 18
D-8120 Weilheim(DE)

(54) **Phosphate von Resorufin-Derivaten, Verfahren zu deren Herstellung sowie deren Verwendung zur Bestimmung der Aktivität von Phosphatasen.**

(57) Beansprucht werden Phosphate von Resorufin-Derivaten der allgemeinen Formeln Ia bzw Ib

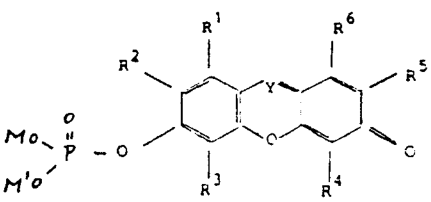

(I a)

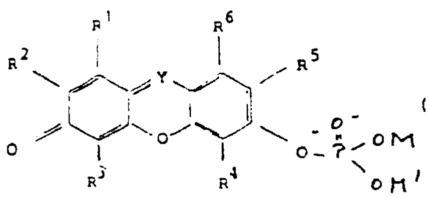

(I b)

in denen

$R^2$ und $R^5$, die gleich oder verschieden sein können, Wasserstoff, Halogen oder eine Niederalkylgruppe,

$R^1$, $R^3$, $R^4$ und $R^6$, die gleich oder verschieden sein können, Wasserstoff, Halogen, eine Cyano-, Niederalkyl-, Niederalkoxy-, Carboxy-, Niederalkoxycarbonyl-, Carboxyniederalkyl-, Niederalkoxycarbonyl-niederalkyl- oder eine gegebenenfalls ein- oder zweifach substituierte Carboxamidogruppe oder die Gruppe

$$-COO-(CH_2CH_2O)_n-R^7$$

in der $R^7$ Wasserstoff oder eine Niederalkyl-Gruppe und n eine ganze Zahl von 1 bis 4 bedeuten,

wobei $R^6$ zusätzlich eine Sulfonyl oder Nitrogruppe vorstellen kann,

Y ein Stickstoff oder die Gruppe N O und

M und M', die gleich oder verschieden sein können Wasserstoff, ein Alkali-, Erdalkali- oder Ammoniumion bedeuten.

Diese Verbindungen eignen sich besonders als chromogene und fluorogene Substrate zur Bestimmung der Aktivität von Phosphatasen. Die beanspruchten Phosphate erhält man, durch Phosphorylierung der phenolischen Funktion entsprechender Resorufinderivate.

Ein weiterer Gegenstand der Anmeldung sind diagnostische Mittel zum Nachweis von Phosphatasen, die als chromogene und fluorogene Substrate die beanspruchten Phosphate enthalten.

EP 0 217 371 A2

# Phosphate von Resorufin-Derivaten, Verfahren zu deren Herstellung sowie deren Verwendung zur Bestimmung der Aktivität von Phosphatasen

Die Bestimmung der Aktivität der Phosphatasen hat in den letzten Jahren sowohl in der klinischen Chemie als auch in der Diagnostik an Bedeutung zugenommen. So wird beispielsweise die alkalische Phosphatase in zunehmendem Maße als Indikator-Enzym für Enzymimmunoassays eingesetzt. Die Aktivitätsbestimmung der sauren Phosphatase ist ein wertvolles Mittel zur Frühdiagnose von Prostata-Karzinomen.

Zur Bestimmung wird üblicherweise die phosphatasehaltige Probe mit einem geeigneten Substrat versetzt, welches dann von dem Enzym gespalten wird. In der Regel handelt es sich bei diesen Substraten um Orthophosphorsäure-Monoester. Gemessen wird die nach der Einwirkung des Enzyms freigesetzte alkoholische Komponente. Bevorzugt werden dafür Verbindungen verwendet, die spektroskopisch im sichtbaren oder auch im UV-Bereich sowie fluorimetrisch nachgewiesen werden können.

So wird beispielsweise in der klinischen Analytik schon lange und häufig 4-Nitrophenylphosphat eingesetzt, welches durch Phosphatasen in 4-Nitrophenol und Phosphat gespalten wird. Die Bildung des gelben Phenolat-Ions wird dabei durch Zugabe von Alkali erreicht, was im Falle der Bestimmung der sauren Phosphatase einen zusätzlichen Umpufferungsschritt bedeutet.

Bei einem anderen Verfahren wird 1-Naphthylphosphat verwendet. Das freigesetzte Naphthol muß zur Visualisierung mit Diazoniumsalz zum Azofarbstoff umgesetzt werden (BABSON, A. (1984), Clin. Chem. 30, 1418).

Die Bestimmung von Enzymaktivitäten mit fluorogenen Substraten ist weit verbreitet. Die Empfindlichkeit gegenüber den photometrischen Methoden ist oft um Zehnerpotenzen erhöht. In manchen Fällen muß mit fluorogenen Substraten gearbeitet werden, so z. B. bei der Untersuchung enzymatischer Aktivitäten in Zellen mit automatischen Geräten, zur Zelldifferenzierung (Zytofluorometrie) oder bei der Analyse immobilisierter Enzyme mittels Durchflußmikrofluorometrie. In anderen Fällen, beispielsweise bei der Bestimmung der enzymatischen Markierung von Testsystemen (Enzymimmunoassays) wird der Multiplikationseffekt der enzymatischen Katalyse durch Verwendung fluorogener Substrate beträchtlich verstärkt.

Bisher bekannte fluorogene Substrate für Phosphatasen besitzen als Fluorophor z. B. Derivate des Indoxyls [HORWITZ, J. P. et al., J. Med. Chem. 9, 447 (1966)] oder des 4-Methylumbelliferons [FERNLEY, H. N. und WALKER, P. E., Biochem. J. 97, 95 (1965)]. Für die kinetische Analyse komplexer Systeme besitzen diese Verbindungen jedoch Nachteile. So unterliegen Indoxyl-Derivate nach ihrer enzymatischen Spaltung einer Reihe chemischer Umwandlungen, die die Analyse komplizieren. 4-Methylumbelliferon-Derivate müssen im UV-Bereich angeregt werden, wobei die Eigenfluoreszenz von gleichzeitig anwesenden biologischen oder synthetischen Materialien stört.

Es bestand daher weiterhin Bedarf an Substraten, mit denen verschiedene Phosphatasen (Phosphomonoester-Hydrolasen) auf einfache, schnelle und zuverlässige Weise bestimmt werden können und die möglicherweise sowohl bei photometrischen als auch fluorometrischen Bestimmungsverfahren eingesetzt werden können. Diesen Bedarf zu erfüllen, war Aufgabe der vorliegenden Erfindung.

Gelöst wird diese Aufgabe durch die neuen Phosphate von Resorufin-Derivaten, die sich mit Hilfe von Phosphatasen in den Phosphorsäureteil und in die Resorufin-Derivate spalten lassen. Letztere sind gut wasserlösliche Verbindungen, die eine gut meßbare Absorption im sichtbaren Bereich aufweisen und sich ferner leicht zur Fluoreszenz anregen lassen.

Gegenstand der vorliegenden Erfindung sind demnach Phosphate von Resorufin-Derivaten der allgemeinen Formel I a bzw. I b

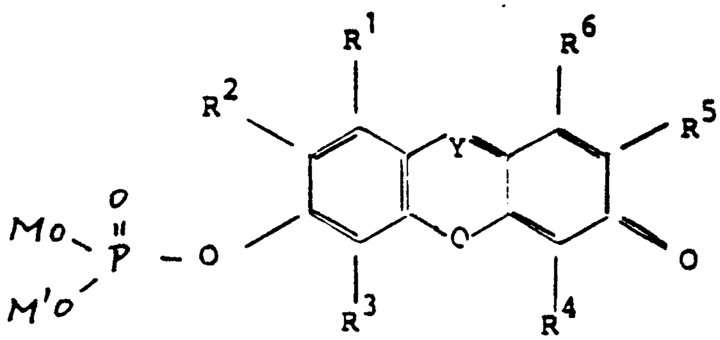

(I a)

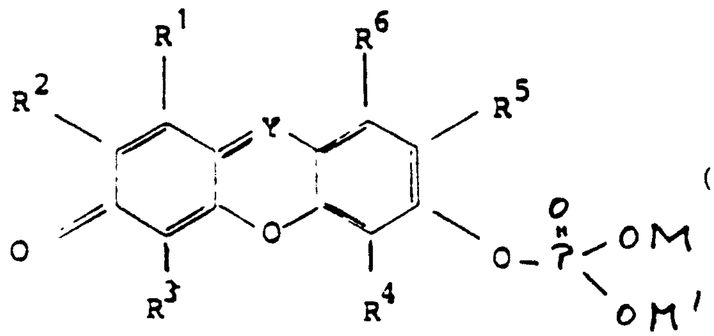

(I b)

in denen

$R^2$ und $R^5$, die gleich oder verschieden sein können, Wasserstoff, Halogen oder eine Niederalkylgruppe,

$R^1$, $R^3$, $R^4$ und $R^6$, die gleich oder verschieden sein können, Wasserstoff, Halogen, eine Cyano-, Niederalkyl-, Niederalkoxy-, Carboxy-, Niederalkoxycarbonyl-, Carboxyniederalkyl-, Niederalkoxycarbonyl-niederalkyl- oder eine gegebenenfalls ein- oder zweifach substituierte Carboxamidogruppe oder die Gruppe

$-COO-(CH_2CH_2O)_n-R^7$,

in der $R^7$ Wasserstoff oder eine Niederalkyl-Gruppe und n eine ganze Zahl von 1 bis 4 bedeuten,
wobei $R^6$ zusätzlich eine Sulfonyl oder Nitrogruppe vorstellen kann, und

Y ein Stickstoff oder die Gruppe $N \rightarrow O$ und
M und M', die gleich oder verschieden sein können, Wasserstoff, ein Alkali-, Erdalkali- oder Ammonium-Ion

bedeuten.

Phosphate von Resorufin-Derivaten der allgemeinen Formel I sind neue Verbindungen. Sie können nach an sich bekannten Methoden durch Phosphorylierung der phenolischen Funktion hergestellt werden.

Vorzugsweise werden in an sich bekannter Weise Resorufin-Derivate der allgemeinen tautomeren Formeln II a und II b

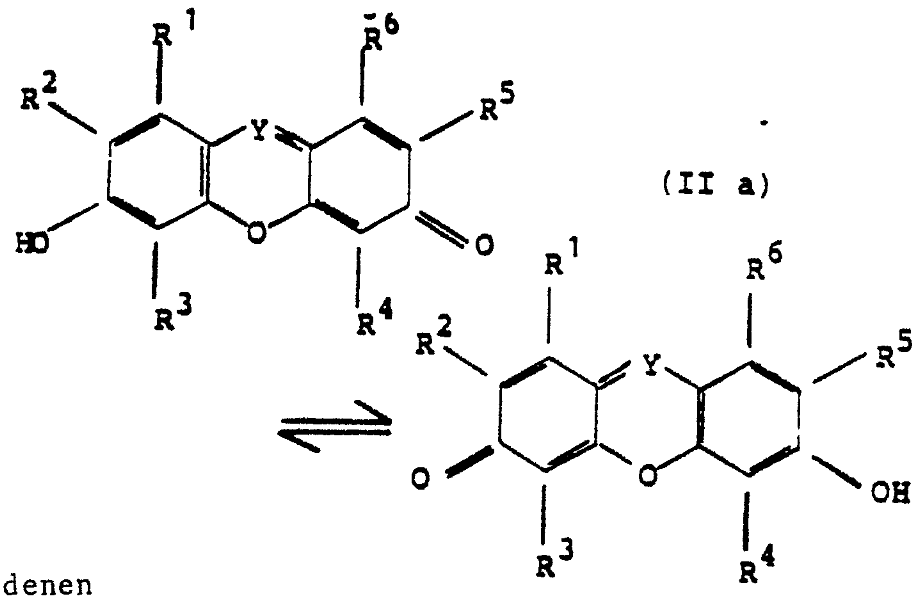

(II a)

(II b)

in denen

$R^1$ bis $R^6$ und Y die oben angegebene Bedeutung haben,

mit einem Halogenid des 5-wertigen Phosphors (vorzugsweise in Gegenwart eines anorganischen oder organischen Säurefängers) umsetzt und die erhaltene Dihalogenphosphonyloxy-Verbindung anschließend hydrolysiert.

Resorufin-Derivate der allgemeinen Formeln II a bzw. II b sind Gegenstand der Europäischen Patentanmeldung 0 156 347.

Als Halogenide des 5-wertigen Phosphors kommen beispielsweise Phosphoroxidtrichlorid, Phosphorpentachlorid oder Pyrophosphorsäuretetrachlorid in Betracht. Das letztgenannte wird bevorzugt eingesetzt.

Als Säurefänger eignen sich anorganische und organische Basen, beispielsweise Alkalicarbonate und Trialkylamine. Als besonders günstig erweist sich die Verwendung von cyclischen Diaminen, wie z. B. Diaza-bicyclo-undecen (DBU), welche durch Ausbildung entsprechender Ammoniumsalze eine besonders gute Löslichkeit der Resorufin-Derivate in den zur Reaktion eingesetzten Lösungs- und Verdünnungsmitteln bewirken.

Als Lösungs- und Verdünnungsmittel eignen sich unter den Reaktionsbedingungen vor allem organische Flüssigkeiten, wie Chloroform, Dichlormethan oder Acetonitril.

Die Reaktion wird vorzugsweise in Gegenwart eines Lösungs- oder Verdünnungsmittels im Temperaturbereich von -30 bis +20° C durchgeführt.

Die Hydrolyse der intermediär gebildeten Dichlorphosphonyloxy-Verbindungen geschieht schonend bei Temperaturen von 0 bis 20°C durch Neutralisation mit wässriger Alkalicarbonat-Lösung, Erdalkalihydroxid-Lösung, Triethylamin oder Triethylammoniumbicarbonat. Die erhaltenen Alkali-, Erdalkali- oder Ammoniumsalze konzentriert man im Vakuum und gewinnt daraus durch geeignete chromatographische Verfahren die gewünschten Phosphorsäure- Resorufinderivat-monoester. Eine andere Methode besteht in der Ausfällung der freien, relativ schwerlöslichen freien Phosphorsäureester aus konzentrierter wässriger Alkali-, Erdalkali- oder Ammoniumsalzlösung durch Ansäuern mit Mineralsäuren, wie z.B. Salzsäure.

Bei der Synthese von Phosphaten der Resorufin-Derivaten der allgemeinen Formel II entstehen in Form ihrer Alkali- oder Ammoniumsalze gut wasserlösliche, nichtfluoreszierende Verbindungen der allgemeinen Formel I, aus denen durch enzymatische Spaltung mit Hilfe von Phosphatasen wieder fluoreszierende Resorufin-Derivate freigesetzt werden.

Unter Halogen in der Definition von $R^1$ bis $R^7$ ist Fluor, Chlor, Brom und Iod, vorzugsweise Chlor und Brom, zu verstehen.

Die "Niederalkylgruppe" in der Definition von $R^1$ bis $R^7$ enthält 1 bis 5, vorzugsweise 1 bis 3 Kohlenstoffatome, wobei die Methylgruppe besonders bevorzugt ist.

Die "Niederalkoxygruppe" in der Definition von $R^4$ und $R^6$ enthält 1 bis 5, vorzugsweise 1 bis 3 Kohlenstoffatome, wobei die Methoxygruppe besonders bevorzugt ist.

Die "Niederalkoxy-" bzw. die "Niederalkyl-"Reste der Niederalkoxycarbonyl-, Carboxy-niederalkyl- sowie der Niederalkoxycarbonyl-niederalkyl-Reste in der Definition der Substituenten $R^4$ und $R^6$ weisen ebenfalls 1 bis 5, vorzugsweise 1 bis 3 Kohlenstoffatome auf, wobei die Methoxy-Gruppe bzw. der Methyl-Rest besonders bevorzugt sind.

Als Substituenten der Carboxamidogruppe kommen Alkyl-, Alkoxyalkyl-, Carboxyalkyl- und Alkoxycarbonylalkylreste in Frage, wobei die Alkyl- oder Alkoxyreste jeweils 1 bis 5, vorzugsweise 1 bis 3 Kohlenstoffatome aufweisen. Bei einer zweifach substituierten Carboxamidfunktion können die beiden Substituenten zu einem Ring geschlossen sein, der durch Heteroatome, z. B. Sauerstoff, Stickstoff und Schwefel, unterbrochen sein kann.

Unter Alkaliionen in der Definition von M und M' sind vor allem Lithium-, Natrium- und Kaliumionen zu verstehen. Erdalkaliionen sind vor allem Magnesium-, Calcium- oder Bariumionen.

Ammoniumionen in der Definition von M und M' können das unsubstituierte Ammoniumion oder durch Alkyl- oder Aralkylreste ein- oder mehrfach substituierte Ammoniumionen sein. Hierbei ist unter Alkylrest ein Rest aus 1 - 5 Kohlenstoffatomen zu verstehen. Bevorzugt sind der Methyl- oder Ethylrest. Unter einem Aralkylrest ist vor allem der Benzylrest zu verstehen. Die Substituenten substituierter Ammoniumionen können sowohl gleich als auch verschieden sein.

Anregung und Emission der erfindungsgemäßen Produkte liegen im sichtbaren Spektralbereich bei hinreichender Quantenausbeute. Die maximale Fluoreszensintensität des Resorufins wird bei pH-Werten über 7,0 erreicht und fällt zu niedrigeren pH-Werten nur langsam ab. Die Glycoside des Resorufins sind in wäßriger Lösung meist gelb gefärbt ($\lambda_{max.}$ bei ca. 470 nm). Nach der enzymatischen Reaktion weisen die Produkte meist eine rote Farbe auf ($\lambda_{max.}$ bei ca. 570 nm), so daß die Substanzen ebenfalls ausgezeichnet für photometrische Bestimmungen und nicht-instrumentelle, visuelle Verfahren geeignet sind.

Ferner werden diagnostische Mittel zur Bestimmung der Aktivität von Phosphatasen beansprucht, die die neuen Phosphate von Resorufin-Derivaten der allgemeinen Formel I enthalten. Die Verwendung der Phosphate von Resorufin- Derivaten der allgemeinen Formel I als Substrate für Phosphatasen führt zu deutlich empfindlicheren Testsystemen, als sie bisher bekannt sind. Die neuen Substrate können zur Bestimmung der Aktivität von Phosphatasen mit Vorteil sowohl im biochemischen, biotechnologischen als auch im klinisch-chemischen Bereich eingesetzt werden. Sie sind empfindlicher. Daraus ergeben sich mehrere Vorteile:

a) Es können geringere Enzym-Aktivitäten gemessen werden.
b) Es können kleinere Probenmengen eingesetzt werden.
c) Die Bestimmung der Aktivität kann in erheblich kürzerer Zeit erfolgen.
d) Der geringe Probeneinsatz und die günstige Meßwellenlänge vermindern außerdem die Störanfälligkeit der Methode durch andere Probenbestandteile.
e) Es kann der Umsatz in Trägermatrizen mit immobilisiertem Enzym gemessen werden.

Es hat sich gezeigt, daß die beanspruchten Substrate zur Bestimmung der Aktivität von Phosphatasen jeglicher Herkunft geeignet sind. Die erfindungsgemäßen diagnostischen Mittel mit Substraten der allgemeinen Formel I reagieren deutlich empfindlicher als die bisher bekannten Testmittel.

Geeignet sind die Phosphate von Resorufin-Derivaten der Formel I auch für immunologische Bestimmungsmethoden, bei denen Phosphatasen als Indikator-Enzyme verwendet werden, deren Aktivität nach Durchführung der immunologischen Reaktion ermittelt werden muß. Solche immunologischen Bestimmungsmethoden mit enzymatischer Indikatorreaktion sind dem Fachmann als Enzymimmunoassays bekannt. Diese Methoden dienen zur Bestimmung der Konzentration von Proteinen, Polysacchariden, Hormonen, Arzneistoffen und anderen nieder-molekularen Substanzen im Bereich von $10^{-5}$ bis $10^{-12}$ mol/l. Je nach Erfordernis von Phasentrennschritten unterscheidet man zwischen homogener und heterogener Testführung. Eine weitere Unterteilung kann in kompetitive und nicht-kompetitive Testprinzipien erfolgen.

Alle Testprinzipien arbeiten jedoch mit Enzym-Antigen- bzw. Enzym-Antikörper-Konjugaten. Die enzymatische Indikatorreaktion ist allen Enzymimmunoassays gemeinsam.
Für solche Zwecke geeignete Indikatorenzyme sind beispielsweise Phosphatasen. Die Bestimmung der Phosphatasen in solchen Enzymimmunoassays erfolgt üblicherweise, indem ein geeignetes Substrat zugesetzt wird, das enzymatisch gespalten und in üblicher Weise photometrisch oder auch fluorometrisch vermessen wird.

Eine Verbesserung des Phosphatase-Testsystems führt demnach ebenfalls zu erheblichen Vorteilen bei solchen Enzymimmunoassays:

1. Die höhere Empfindlichkeit ermöglicht auch hier eine weitere Erniedrigung der Nachweisgrenzen, kürzere Reaktionszeiten und geringeren Probeneinsatz und damit auch geringere Störungen durch andere Probenbestandteile.

2. Die günstigere Meßwellenlänge vermindert bei bestimmten Reaktionsführungen die Störanfälligkeit der Methode durch unlösliche Bestandteile, beispielsweise durch Trübungen.

Das diagnostische Mittel enthält neben einem oder mehreren der erfindungsgemäßen Substrate der allgemeinen Formel I, ein geeignetes Puffersystem sowie gegebenenfalls weitere geeignete, üblicherweise für solche diagnostische Mittel verwendete Zusatzsstoffe, wie beispielsweise Netzmittel, Stabilisatoren usw. Das diagnostische Mittel kann in Form einer Lösung, als Lyophilisat, als Pulvergemisch, Reagenztablette oder auf einem saugfähigen Träger aufgezogen, vorliegen.

Das erfindungsgemäße diagnostische Mittel in Form einer Lösung enthält vorzugsweise sämtliche für den Test benötigten Reagentien. Als Lösungsmittel kommen Wasser oder Gemische von Wasser mit einem wasserlöslichen organischen Lösungsmittel, wie z. B. Methanol, Äthanol, Aceton oder Dimethylformamid, in Frage. Aus Haltbarkeitsgründen kann es vorteilhaft sein, die für den Test benötigten Reagentien auf zwei oder mehr Lösungen zu verteilen, die erst bei der eigentlichen Untersuchung vermischt werden.

Zur Herstellung des diagnostischen Mittels in Form eines Lyophilisats im Gesamtgewicht von jeweils etwa 5 - 20 mg, vorzugsweise etwa 10 mg, wird eine Lösung getrocknet, die neben sämtlichen für den Test benötigten Reagentien übliche Gerüstbildner, wie z. B. Polyvinylpyrrolidon, und eventuelle weitere Füllstoffe, wie z. B. Mannit, Sorbit oder Xylit enthält.

Ein diagnostisches Mittel in Form von Pulvermischungen oder Reagenztabletten läßt sich herstellen, indem die Bestandteile des Testes mit üblichen galenischen Zusatzstoffen versetzt und granuliert werden. Zusatzstoffe dieser Art sind z. B. Zuckeralkohole, wie Mannit, Sorbit oder Xylit, oder andere lösliche inerte Verbindungen, wie Polyethylenglykole oder Polyvinylpyrrolidon. Die Pulvermischungen oder Reagenztabletten weisen im allgemeinen ein Endgewicht von ungefähr 30 bis 200 mg, vorzugsweise 50 bis 80 mg, auf.

Zur Herstellung des diagnostischen Mittels in Form eines Teststreifens wird ein saugfähiger Träger, vorzugsweise Filterpapier, Cellulose oder Kunstfaservlies mit Lösungen der erforderlichen, üblicherweise zur Herstellung von Teststreifen verwendeten Reagentien in leicht flüchtigen Lösungsmitteln, wie z. B. Wasser, Methanol, Äthanol oder Aceton imprägniert. Dies kann in einem Imprägnierschritt erfolgen. Oft ist es jedoch zweckmäßig, die Imprägnierung in mehreren Schritten durchzuführen, wobei Lösungen eingesetzt werden, die jeweils einen Teil der Bestandteile des diagnostischen Mittels enthalten. So kann beispielsweise in einem ersten Schritt mit einer wäßrigen Lösung, die den Puffer und andere wasserlösliche Zusatzstoffe enthält, und dann in einem zweiten Schritt mit einer Lösung, die das Phosphatase-Substrat enthält, imprägniert werden. Die fertigen Testpapiere können als solche verwendet werden oder in an sich bekannter Weise an Griffen angeklebt oder vorzugsweise zwischen Kunststoffen und feinmaschigen Netzwerken gemäß DBP 2118455 eingesiegelt werden.

Die nachfolgenden Beispiele zeigen einige der zahlreichen Verfahrensvarianten, die zur Synthese der erfindungsgemäßen Verbindungen beschritten werden können sowie beispielhaft die Verwendung der neuen Phosphate von Resorufin-Derivaten zur Bestimmung der Aktivität von Phosphatasen. Sie sollen jedoch nicht eine Einschränkung des Erfindungsgegenstandes bedeuten.

Beispiel 1

Resorufin-phosphorsäure

5 g (23,5 mMol) Resorufin werden in 200 ml Dichlormethan suspendiert und mit 7,5 ml (49,3 mMol) 1,8-Diazabicyclo-[5,4,0]-undec-7-en versetzt. Man rührt das Gemisch bei Raumtemperatur bis zur vollständigen Lösung und tropft diese dann unter Rühren innerhalb von ca. 1 Stunde in ein auf etwa -15° C gekühltes Gemisch aus 15 ml (95 mMol) Pyrophosphorsäurechlorid in 500 ml Dichlormethan.

Das Reaktionsgemisch wird anschließend in 500 ml einer gesättigten wässrigen Natriumhydrogencarbonat-Lösung eingerührt und solange mit zusätzlichem Natriumhydrogencarbonat versetzt, bis keine Kohlendioxid-Bildung mehr zu beobachten ist. Nach Abtrennung der wässrigen Phase und Konzentrieren derselben im Vakuum auf ca. 200 ml Volumen wird mit 5 N Salzsäure auf einen pH-Wert von 2,5 eingestellt. Bei diesem pH-Wert scheidet sich die freie Resorufinphosphosäure als feiner, gelborange gefärbter Niederschlag ab. Nach Trocknung im Vakuum über Calciumchlorid werden 1,25 g

(18 % der Theorie) an Resorufinphosphosäure erhalten.

Elementaranalyse: $C_{12}H_8NO_6P$ x $H_2O$, Molekulargewicht 311,2

C (berechnet): 46,3 %, C (gefunden): 44,8 %
H (berechnet): 3,2 %, H (gefunden): 2,8 %
N (berechnet): 4,5 %, N (gefunden): 4,5 %
P (berechnet): 10,0 %, P (gefunden): 9,6 %

Elektrophoretische Mobilität (Papier, 0,05 M Triethylammoniumbicarbonatpuffer, pH 7,5), relativ zu Resorufin (= 1): 4,5

Rf-Wert (DC auf Cellulose CEF, Riedel de Haen, Fließmittel: wässrige 1 M Ammoniumacetat-Lösung/Ethanol = 5:2): 0,18

## Beispiel 2

### Resazurin-phosphorsäure

251 mg (1 mMol) Resazurin werden in 25 ml trockenem Ether suspendiert und das Gemisch auf -30° C gekühlt. Dazu tropft man innerhalb von ca. 20 Minuten 2,5 g (10 mMol) Pyrophosphosäurechlorid und rührt danach weitere 60 Minuten im Kältebad. Anschließend werden zur Reaktionslösung etwa 50 ml Ether zugefügt, wobei sich ein braungelber Niederschlag abscheidet. Dieser wird mehrmals mit frischem Ether digeriert, dann durch Zugabe von Eis, welchem 0,5 ml Triethylamin zugefügt wurden, hydrolysiert.

Zur Abtrennung nicht umgesetzten Resazurins wurde die wässrige Lösung auf einen Ionenaustauscher DEAE-Sephadex A-25 in der Carbonatform gegeben (20 ml Säulenvolumen) und mit einem linearen Gradienten Wasser auf 0,5 M wässrige Triethylammoniumcarbonat-Lösung eluiert.

Die produktenthaltenden Fraktionen werden vereinigt, im Vakuum eingedampft, der Rückstand mehrfach mit Methanol aufgenommen und wiederkonzentriert. Nach letztlichem Lösen in Wasser und Lyophilisation werden 40 mg Resazurin-phosphorsäure in Form des Triethylammoniumsalzes erhalten.

Elektrophoretische Mobilität (Papier,
0,05 M Triethylammoniumbicarbonatpuffer, pH 7,5)
relativ zu Resazurin (= 1): 4,1

Rf-Wert: (DC auf Cellulose CEF, Riedel de Haen,
Fließmittel 1 M wässrige Ammoniumacetatlösung/Ethanol = 5:2)
0,2

Beispiel 3

Resorufin-4-carbonsäuremorpholid-phosphat

a) Resazurin-4-carbonsäure

1,60 g (10,5 mmol) Nitrosoresorcin, 1,55 g (10,0 mmol) 2,6-Dihydroxybenzoesäure und 0,86 g (10 mmol) Braunstein werden in 20 ml Methanol aufgenommen und auf 0° C gekühlt. Dazu werden 1,06 ml konz. Schwefelsäure zugetropft. Es wird noch 2 Stunden ohne Kühlung weitergerührt. Das ausgefallene rote Produkt wird abfiltriert, mit Methanol gewaschen und getrocknet. Ausbeute: 2,3 g (85 % der Theorie) Resazurin-4-carbonsäure.

UV/VIS (0,1 M Kaliumphosphatpuffer pH 7,5):
$\lambda_{max}$= 614 nm ($\varepsilon$ = 48 $cm^2$ $mol^{-1}$)
Nach Ansäuern: $\lambda_{max}$= 522 nm ($\varepsilon$= 32 $cm^2$ $mol^{-1}$)

b) Resorufin-4-carbonsäure

2,3 g Resazurin-4-carbonsäure werden in 20 ml Wasser und 5 ml 25 %igem Ammoniak gelöst. Zu der blauen Lösung werden unter Eiskühlung 5 g Zinkstaub gegeben. Danach wird die Eiskühlung entfernt, so daß sich die Lösung allmählich auf Raumtemperatur erwärmt. Die Reduktion läßt sich leicht am Farbumschlag von blau nach dunkelviolett erkennen bzw. mit Hilfe der Dünnschicht-Chromatographie (Fließmittel: Methanol/Essigester 1:1) beobachten. Das überschüssige Zinkpulver wird abfiltriert. Die Reaktionslösung wird mit 5 ml Eisessig und konz. Salzsäure angesäuert. Das ausgefallene Produkt wird abfiltriert, mit verdünnter Salzsäure gewaschen und im Vakuum über Calciumchlorid getrocknet. Ausbeute: 1,8 g (82 % der Theorie) Resorufin-4-carbonsäure.

UV/VIS: (0,1 M Kaliumphosphatpuffer, pH 7,5):
$\lambda_{max}$= 579,4 nm ($\varepsilon$ = 48,6 $cm^2$ $mol^{-1}$;
Nach Ansäuern $\lambda_{max}$= 485,9 nm ($\varepsilon$ = 34,7 $cm^2$ $mol^{-1}$.
Fluoreszenz: Absorption: $\lambda_{max}$= 579 nm
Emission: $\lambda_{max}$= 593 nm

c) N,O,O-Triacetyldihydroresorufin-4-carbonsäure

5 g (19,4 mmol) Resorufin-4-carbonsäure oder 5,3 g (19,4 mmol) Resazurin-4-carbonsäure werden in 100 ml 10 %iger Salzsäure 0,5 Stunden mit 7 g (38 mmol) Zinn-II-chlorid unter Rückfluß erhitzt. Dabei färbt sich die Lösung grün. Man läßt erkalten, filtriert die ausgefallene Dihydroresorufin-4-carbonsäure unter Stickstoff ab und trocknet im Vakuum über Phosphorpentoxid. Das so erhaltene Rohprodukt wird 30 min. mit 30 ml Acetanhydrid und 20 mg Natriumacetat unter Rückfluß erhitzt. Man gibt das Reaktionsgemisch auf 200 ml Eiswasser und rührt 14 Stunden. Der Niederschlag wird aus Ethanol/Wasser umkristallisiert. Man erhält 4,8 g (65 %) N,O,O-Triacetyldihydroresorufin-4-carbonsäure.

Schmelzpunkt: 197 - 199° C
DC (Kieselgel, Fließmittel Chloroform/Methanol/Eisessig 9:1:0,1),
Rf = 0,33

d) N,O,O-Triacetyldihydroresorufin-4-carbonsäurechlorid

3,85 g (10 mmol) N,O,O-Triacetyldihydroresorufin-4-carbonsäure werden mit 5,4 ml (60 mmol) Oxalylchlorid versetzt und auf -10° C gekühlt. Dazu gibt man einen Tropfen Dimethylformamid und läßt das Reaktionsgemisch unter Rühren auf Raumtemperatur erwärmen. Das Edukt löst sich dabei unter Gasentwicklung. Nach Beendigung der Gasentwicklung rührt man noch 0,5 Stunden, dampft ein, nimmt je dreimal mit 20 ml trockenen Methylenchlorid auf und dampft bis zur Trockne ein. Man erhält so 4 g rohes Säurechlorid, das ohne weitere Reinigung weiterverarbeitet wird.

DC (Kieselgel, Fließmittel Chloroform/Methanol/Eisessig 9:1:0,1)
Rf = 0,42;
farbloser Fleck, der sich nach einigen Stunden rot färbt.

e) N,O,O-Triacetyldihydroresorufin-4-carbonsäure-morpholid

11,3 g (31,4 mmol) rohes Säurechlorid werden in 150 ml trockenem Methylenchlorid gelöst. Dazu tropft man 8,7 ml (63 mmol) Triethylamin und anschließend 3,3 ml (37,7 mmol) Morpholin. Man rührt noch 2 Stunden, wäscht die Lösung mit 1 %iger Citronensäure, Natriumhydrogencarbonat-Lösung und Wasser, trocknet die organische Phase über Magnesiumsulfat und dampft ein. Der Rückstand wird aus Ethanol kristallisiert.

Ausbeute: 8,1 g (63 %), Schmelzpunkt: 133 - 135° C (Zersetzung)

f) Resorufin-4-carbonsäure-morpholid

3,7 g (9 mmol) Triacetyldihydroresorufin-4-carbonsäure-morpholid werden mit 250 ml Methanol und 250 ml Wasser aufgenommen. Dazu gibt man 36 ml 1 n Natronlauge und 6,0 g (18 mmol) Kaliumhexacyanoferrat-III und rührt 14 Stunden bei Raumtemperatur. Nach Ansäuern mit Salzsäure auf pH 3 dampft man zur Trockne ein und digeriert mit Aceton. Die Farbstoff-Lösung wird über 500 ml Kieselgel mit Aceton als Fließmittel filtriert. Nach Eindampfen des farbstoffhaltigen Eluats erhält man 2,3 g (80 %) Resorufin-4-carbonsäure-morpholid.

UV-VIS (0,1 M Kaliumphosphat-Puffer, pH 7,5):
$\lambda_{max}$ = 575 nm, $\varepsilon$ = 55.000 $cm^2$ $mol^{-1}$.
DC (Fließmittel, siehe unter d)
Rf = 0,52

$^1$H-NMR ($[D]_6$-DMSO): $\delta$ = 3,3 - 3,8 (m, 8 H); 6,50 (d, J = 2 Hz, 1 H); 6,64 (d, J = 10 Hz, 1 H); 6,76 (dd, J = 10 und 2 Hz, 1 H); 7,44 und 7,51 (jeweils d, J = 10 Hz, 2 H).

g) Resorufin-4-carbonsäuremorpholid-phosphat

Resorufin-4-carbonsäuremorpholid-phosphat wurde durch Umsetzung von Resorufin-4-carbonsäuremorpholid mit Pyrophosphorsäurechlorid analog Beispiel 1 dargestellt. Die Aufreinigung erfolgte durch präparative high pressure liquid chromatography (HPLC) an einer RP-C 18-Säule mit einem Isopropanol/Triethylammoniumacetat-Gradient.

Elektrophoretische Mobilität (Papier,
0,05 M Triethylammoniumbicarbonatpuffer, pH 7,5)
relativ zu Resorufin-4-carbonsäuremorpholid (=1) : 1,9

Rf-Wert: (DC auf Cellulose CEF, Riedel de Haen,
Fließmittel 1 M wässrige Ammoniumacetat-Lösung/Ethanol = 5:2):
0,3

Beispiel 4

2,8-Dibrom-resorufin-phosphat

Das Produkt wurde durch Umsetzung von 2,8-Dibrom-resorufin (AFANASIEVA, G. B. et al., Khim. Geterotsik L. Soedin. (1974), S. 348 - 353) mit Pyrophosphorsäurechlorid analog Beispiel 1 erhalten.

Elektrophoretische Mobilität (Papier,
0,05 M Triethylammoniumbicarbonatpuffer, pH 7,5)
relativ zu 2,8-Dibrom-resorufin (= 1) : 3,2

Rf-Wert: (DC auf Cellulose CEF, Riedel de Haen, Fließmittel
1 M wässrige Ammoniumacetat-Lösung/Ethanol = 5:2):
0,28

## Beispiel 5

### 4-Methyl-resorufin-phosphat

4-Methyl-resorufin

32,5 g (0,6 mol) Kaliumhydroxid in 300 ml Ethanol gelöst, werden mit 50 g (0,4 mol) 2-Methyl-resorcin versetzt und bis zur vollständigen Lösung gerührt. Zu der mit Eis-Kochsalz gekühlten Lösung werden langsam 63,5 ml (0,43 mol) Isopentylnitrit zugetropft, so daß die Innentemperatur von +10° C nicht überschritten wird. Nach erfolgtem Zutropfen wird noch weitere zwei Stunden im Eisbad gerührt. Der abgeschiedene Feststoff wird abgesaugt, mit Ethanol gewaschen und anschließend in 270 ml Wasser gelöst und durch Zugabe von konzentrierter Salzsäure wieder ausgefällt. Der erhaltene gelbe Niederschlag wird abgesaugt und mit Wasser gewaschen. Das Produkt wird bei 50° C über Calciumchlorid im Vakuum getrocknet. Man erhält 75 g 4-Nitroso-2-methyl-resorzin.

75 g 4-Nitroso-2-methyl-resorzin und 42 g (0,38 mol) Resorcin werden in 750 ml Methanol gelöst. Zu dieser Lösung werden unter Eiskühlung 36 g Mangandioxid zugegeben. Anschließend werden 43 ml konzentrierte Schwefelsäure zugetropft und 2 Stunden gerührt. Dann wird vom Ungelösten abgesaugt und das Filtrat im Vakuum auf etwa ein Drittel des Volumens eingeengt. Das Konzentrat wird in 1,5 l Wasser eingerührt. Dabei scheidet sich ein Feststoff ab. Er wird abgesaugt und mit Wasser nachgewaschen. Anschließend wird der Feststoff in 750 ml Wasser gelöst und mit 180 ml 25 %iger Ammoniaklösung und mit 40 g Zinkstaub versetzt. Nach 30 minütigem Rühren wird 4-Methyl-resorufin mit konzentrierter Salzsäure ausgefällt, abgesaugt und bei 50° C über Calciumchlorid im Vakuum getrocknet. Man erhält so 45 g (52 % der Theorie) Produkt.

Rf-Wert (DC auf Cellulose CEF, Riedel de Haen, Fließmittel 1 m wässrige Ammoniumacetat-Lösung/Ethanol = 5:2):

0,71

4-Methyl-resorufin-phosphat wird durch Umsetzung von 4-Methyl-resorufin mit Pyrophosphorsäurechlorid analog Beispiel 1 hergestellt.

Elektrophoretische Mobilität (Papier, 0,05 m Triethylammoniumbicarbonat-Puffer, pH 7,5) relativ zu 4-Methyl-resorufin (= 1): 3,4

Rf-Wert (DC auf Cellulose CEF, Riedel de Haen, Fließmittel 1 m wässrige Ammoniumacetat-Lösung/Ethanol = 5:2):

0,2

Beispiel 6

Bestimmung der Aktivität von alkalischer Phosphatase

Resorufin-4-carbonsäuremorpholid-phosphat wird in einer Konzentration von 1 mg/ml in 0,1 M Glycinpuffer, pH 10,5, gelöst. Bei Zugabe von einer Lösung, die alkalische Phosphatase enthält, erfolgt ein Farbumschlag von orangegelb nach blauviolett.

Die Intensität der Blauviolettfärbung ist der Konzentration der alkalischen Phosphatase in der Probe proportional.

Mit Hilfe von Proben mit bekannter Konzentration an alkalischer Phosphatase läßt sich eine Eichkurve ermitteln, anhand derer der unbekannte alkalische Phosphatase-Gehalt einer Probe bestimmt werden kann.

Beispiel 7

Bestimmung der Aktivität von saurer Phosphatase

Ein Chromatographiepapierstreifen wird mit einer Lösung von 2,8-Dibrom-resorufin-phosphat in einer Konzentration von 1 mg/ml in 0,1 M Citratpuffer, pH 5,0, getränkt und getrocknet. Beim Auftüpfeln einer Lösung mit saurer Phosphatase erfolgt Farbumschlag nach blaurot.

Die Intensität der blauroten Färbung ist der Konzentration der sauren Phosphatase in der Probe proportional.

Mit Hilfe von Proben mit bekannten Konzentrationen an saurer Phosphatase läßt sich eine Eichkurve ermitteln, anhand derer, der unbekannte saure Phosphatase-Gehalt einer Probe bestimmt werden kann.

## Patentansprüche

1. Phosphate von Resorufin-Derivaten der allgemeinen Formeln Ia bzw. Ib

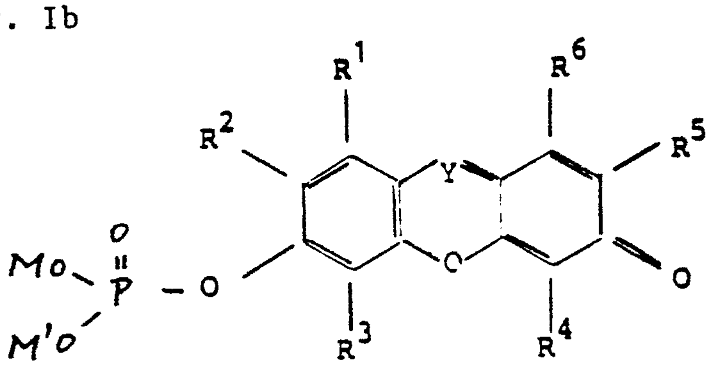

(I a)

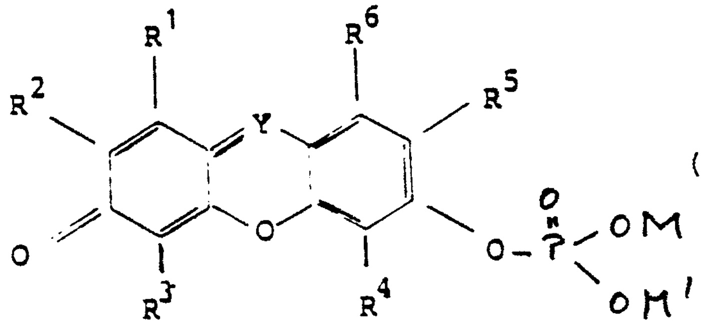

(I b)

in denen

$R^2$ und $R^5$, die gleich oder verschieden sein können, Wasserstoff, Halogen oder eine Niederalkylgruppe
$R^1$, $R^3$, $R^4$ und $R^6$, die gleich oder verschieden sein können, Wasserstoff, Halogen, eine Cyano-, Niederalkyl-, Niederalkoxy-, Carboxy-, Niederalkoxycarbonyl-, Carboxyniederalkyl-, Niederalkoxycarbonyl-niederalkyl- oder eine gegebenenfalls ein- oder zweifach substituierte Carboxamidogruppe oder die Gruppe

$$-COO-(CH_2CH_2O)_n-R^7$$

in der $R^7$ Wasserstoff oder eine Niederalkyl-Gruppe und n eine ganze Zahl von 1 bis 4 bedeuten,
wobei $R^6$ zusätzlich eine Sulfonyl- oder Nitrogruppe vorstellen kann,
Y Stickstoff oder die Gruppe $N \rightarrow O$ und
M und M', die gleich oder verschieden sein können, Wasserstoff, ein Alkali-, Erdalkali- oder Ammoniumion

bedeuten.

0217371

dadurch gekennzeichnet, daß man in an sich bekannter Weise Verbindungen der allgemeinen tautomeren Formel IIa und IIb

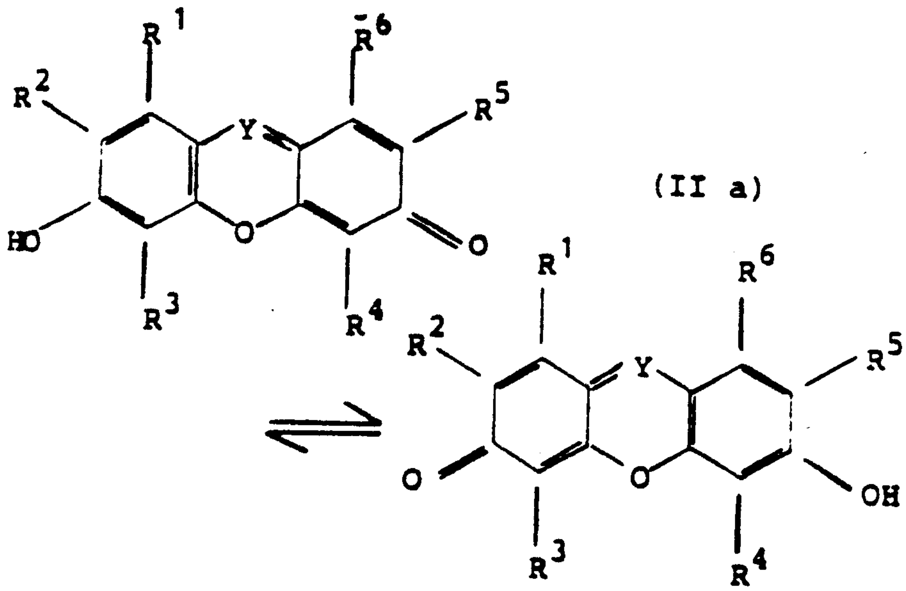

(II a)

(II b)

in denen

$R^1$ bis $R^6$ und Y die oben angegebene Bedeutung haben,

mit einem Halogenid des 5-wertigen Phosphors umsetzt und die als Zwischenprodukt erhaltenen Dihalogenphosphonyloxy-Verbindungen anschließend hydrolysiert.

3. Verwendung der Phosphate von Resorufin-Derivaten gemäß Anspruch 1 zur Bestimmung der Aktivität von Phosphatasen.

4. Diagnostisches Mittel zum Nachweis von Phosphatasen enthaltend ein oder mehrere chromogene oder/und fluorogene Substrate, eine geeignete Puffersubstanz sowie gegebenenfalls weitere üblicherweise verwendete Hilfsstoffe, dadurch gekennzeichnet, daß als chromogene und fluorogene Substrate Phosphate von Resorufin-Derivaten gemäß Anspruch 1 eingesetzt werden.

5. Diagnostisches Mittel gemäß Anspruch 4, dadurch gekennzeichnet, daß als zusätzliche Hilfsmittel Netzmittel, galenische Zusatzstoffe oder/und Gerüstbildner verwendet werden.

2. Verfahren zur Herstellung der Phosphate von Resorufin-Derivaten der allgemeinen Formeln Ia bzw. Ib,

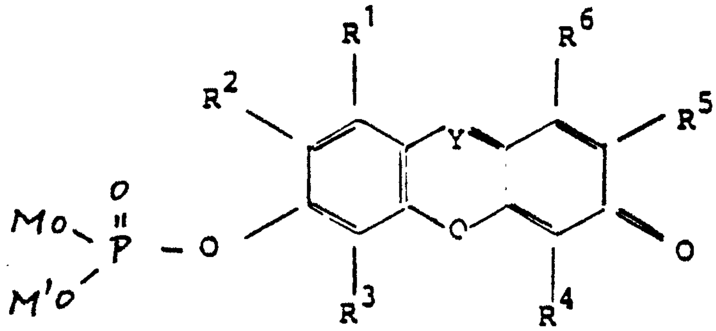

(I a)

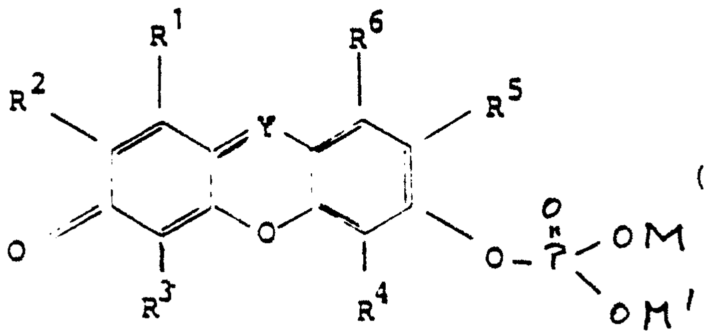

(I b)

in denen

$R^2$ und $R^5$, die gleich oder verschieden sein können, Wasserstoff, Halogen oder eine Niederalkylgruppe,
$R^1$, $R^3$, $R^4$ und $R^6$, die gleich oder verschieden sein können, Wasserstoff, Halogen, eine Cyano-, Niederalkyl-, Niederalkoxy-, Carboxy-, Niederalkoxycarbonyl-, Carboxyniederalkyl-, Niederalkoxycarbonyl-niederalkyl- oder eine gegebenenfalls ein- oder zweifach substituierte Carboxamidogruppe oder die Gruppe

$-COO-(CH_2CH_2O)_n-R^7$

in der $R^7$ Wasserstoff oder eine Niederalkyl-Gruppe und n eine ganze Zahl von 1 bis 4 bedeuten,
wobei $R^6$ zusätzlich eine Sulfonyl- oder Nitrogruppe vorstellen kann, und
Y Stickstoff oder die Gruppe N O und
M und M', die gleich oder verschieden sein können, Wasserstoff, ein Alkali-, Erdalkali- oder Ammoniumion

bedeuten,